# NEUE EUROPÄISCHE PATENTSCHRIFT

(11) **EP 2 788 572 B2**
(45) Veröffentlichungstag und Bekanntmachung des Hinweises auf die Entscheidung über den Einspruch: **09.08.2023**
(45) Hinweis auf die Patenterteilung: 09.11.2016
(21) Anmeldenummer: 12797683.5
(22) Anmeldetag: 04.12.2012
(51) Int. Cl.: E06B 7/23, A01N 25/34, A01N 55/02, A61L 2/238

(54) **TÜRDICHTUNG FÜR EINE SCHWELLENLOSE TÜR**
DOOR SEAL FOR A SILL-FREE DOOR
JOINT D'UNE PORTE SANS SEUIL

(30) Priorität: 06.12.2011 CH 19242011
(43) Veröffentlichungstag der Anmeldung: 15.10.2014
(73) Patentinhaber: ASSA ABLOY (Schweiz) AG, 8805 Richterswil (CH)
(72) Erfinder: DINTHEER, Andreas, 8308 Illnau (CH)
(74) Vertreter: Clerc, Natalia
(86) Internationale Anmeldenummer: PCT/CH2012/000266
(87) Internationale Veröffentlichungsnummer: WO 2013/082729

(56) Entgegenhaltungen:
- EP-A2- 0 338 974
- CH-A- 134 875
- CH-A- 465 830
- DE-U1- 29 820 770
- DE-U1-202010 005 197
- GB-A- 2 442 440

## Beschreibung

### TECHNISCHES GEBIET

Die vorliegende Erfindung betrifft eine Türdichtung für eine schwellenlose Tür gemäss Oberbegriff des Patentanspruchs 1.

### STAND DER TECHNIK

Türdichtungen werden in Bereichen eingesetzt, wo ein Schallschutz und ein Schutz vor Zugluft gewünscht sind. Ebenso sollen sie lichtdicht abschliessen. Einfache Türdichtungen sind sogenannte Schleifdichtungen, deren Dichtlippe oder Bürste eine untere Stirnfläche des Türflügels nach unten überragt.

Ferner sind absenkbare Türdichtungen bekannt, welche üblicherweise im Wesentlichen aus einer nach unten offenen, u-förmigen Führungsschiene, einer in der Führungsschiene gehaltenen und relativ zu dieser verschiebbaren Dichtleiste und einem Antriebsmechanismus zum Absenken bzw. zum Anheben der Dichtleiste bestehen. Üblicherweise senkt sich die Dichtleiste automatisch beim Schliessen der Tür ab, indem ein Betätigungsstab zurückgeschoben wird und den mechanischen Absenkmechanismus in Betrieb setzt. Es ist jedoch auch möglich, einen von Hand betätigbaren Antriebsmechanismus vorzusehen; beispielsweise einen Mechanismus, welcher über die Türklinke aktiviert wird. Türdichtungen mit mechanischen Absenkmechanismen sind beispielsweise aus EP 0 338 974, DE 299 16 090 und EP 0 509 961 bekannt. Diese Türdichtungen haben sich bewährt. Insbesondere die Türdichtung gemäss EP 0 338 974 zeichnet sich dank der einstückigen Dichtlippe, deren Schenkel gleitend an der Führungsschiene anliegen, durch eine besonders effiziente Dichtleistung aus.

Derartige Türdichtungen werden auch in Spitälern, Kliniken, Arztpraxen und vergleichbaren Einrichtungen eingesetzt. In diesen Gebäuden sind die hygienischen Anforderungen sehr hoch, um Infektionen möglichst zu vermeiden. So werden beispielsweise Türgriffe und Böden mit starken Desinfektionsmitteln behandelt, um ein Ausbreiten von Krankheiten zu verhindern. Böden werden dabei richtiggehend überschwemmt. Auch gibt es im Stand der Technik diverse Vorschläge, um Türgriffe möglichst keimfrei zu halten.

DE 103 05 142 schlägt beispielsweise vor, einen Türgriff aus Kunststoff herzustellen, welchem Silberionen beigemischt ist. Des Weiteren wird vorgeschlagen, einen Türgriff aus Metall mit einem derartigen Kunststoff zu beschichten.

DE 20 2004 017 284 beschreibt einen Türklinken-/Hand-Desinfiszierer. Ein Behälter mit Desinfektionsflüssigkeit ist hierfür in der Tür angeordnet.

Den übrigen Teilen der Tür, insbesondere der Türunterseite, wird jedoch kaum Beachtung geschenkt. Dabei können gerade Schleifdichtungen oder Absenkdichtungen Keimherde bilden, wobei durch die Bewegung der Tür beim Öffnen und Schliessen diese Keime über grosse Bereiche des Zimmers verteilt werden. Erschwerend kommt hinzu, dass die Unterseite des Türflügels zudem für die Reinigung schlecht zugänglich ist.

Zwar offenbart GB 486 162 aus dem Jahre 1937 ein Dichtungsmaterial, um Türen und Fenster gegen schädliche Gase, insbesondere Senfgas, abzudichten. Das Material soll zusätzlich zur Abwehr von bakteriellen Angriffen dienen. Dieses Dichtungsmaterial soll jedoch als Schutz vor kriegerischen Angriffen von aussen dienen und ist lediglich als zusätzliche Dichtung auf die bereits vorgesehenen Dichtungen gedacht.

US 2011/0031855 betrifft ferner eine Waschmaschine mit einer Dichtung zwischen Tür und Innenraum, welche antimikrobiell ausgebildet ist.

CH 134 875 beschäftigt sich mit Fugendichtungen. Diese Fugendichtungen sind schnurartige Gebilde, welche in die abzudichtende Fuge eingepresst werden und dort alleine durch Adhäsionswirkung zwischen einer dauernd plastischen und wasserabstossenden Imprägnierungsmasse des Gebildes und der Haftfläche des Türrahmens haften.

GB 2 442 440 beschäftigt sich mit Fingerplatten und Türgriffen, welche antimikrobielle Substanzen enthalten.

### DARSTELLUNG DER ERFINDUNG

Es ist deshalb eine Aufgabe der Erfindung, die Hygiene in Räumen, insbesondere in medizinischen Einrichtungen, zu verbessern.

Diese Aufgabe löst eine Türdichtung für eine schwellenlose Tür mit den Merkmalen des Patentanspruchs 1.

Die erfindungsgemässe Türdichtung für eine schwellenlose Tür ist als automatisch absenkbare Dichtung ausgebildet und weist ein Dichtelement zur Auflage auf einen Boden und zur Dichtung eines Spaltes zwischen Türflügel und Boden auf. Erfindungsgemäss ist mindestens ein Teil des Dichtelements keimhemmend oder keimtötend wirkend ausgebildet.

Unter keimtötend wird verstanden, dass Keime, wie zum Beispiel Bakterien, Viren oder Pilze, absterben. Keimhemmend bedeutet, dass eine Reproduktion und somit eine Vermehrung der Keime verhindert wird und/oder dass sich die Keime nicht auf das Material der Dichtung ablagern können.

Vorzugsweise ist das gesamte Dichtelement keimhemmend oder keimtötend wirkend ausgebildet. Erfindungsgemäss ist mindestens derjenige Teil des Dichtelements keimhemmend oder keimtötend ausgebildet, welcher dichtend auf dem Boden aufliegt. Das Dichtelement kann beispielsweise eine ein- oder mehrteilige Dichtlippe, insbesondere aus Silikon, oder eine Bürste sein.

Erfindungsgemäss ist das Dichtelement relativ zum Gehäuse automatisch anhebbar oder absenkbar. Das Dichtelement ist vorzugsweise in einer Trägerschiene angeordnet, welche in einem Gehäuse automatisch anhebbar und absenkbar befestigt ist.

Ist das Dichtelement in einem Gehäuse und/oder an einer Trägerschiene angeordnet, so können auch diese Elemente keimhemmend oder keimtötend ausgebildet sein. In einer Ausführungsform sind sämtliche Teile der Dichtung keimhemmend oder keimtötend ausgebildet.

Vorzugsweise ist die Türdichtung eine automatisch absenkbare Dichtung mit einem federbelasteten Auslöseknopf, welcher dem Gehäuse der Dichtung einseitig vorsteht und beim Schliessen des Türflügels am Türrahmen ansteht und so den federbelasteten mechanischen Auslösemechanismus betätigt und die Dichtleiste absenkt. Vorzugsweise ist auch dieser Auslöseknopf keimhemmend oder keimtötend ausgebildet. Werden andere Arten von mechanischen Auslösungen verwendet, so sind auch diese vorzugsweise keimhemmend oder keimtötend ausgebildet.

In einem bevorzugten Ausführungsbeispiel umfasst die keimhemmende oder keimtötende Ausbildung eine Beschichtung und/oder Imprägnierung mit keimhemmenden oder keimtötenden Mitteln. Beispielsweise kann die Beschichtung eine Beschichtung mit Silberionen sein. Die Beschichtung kann auch eine Kupferverbindung, Silber oder Silberverbindungen aufweisen. Die keimhemmende oder keimtötende Ausbildung ist dauerhaft. Die hierfür notwendigen Bestandteile lassen sich dem Material beimischen oder als Zusätze beigeben. Sie lassen sich auch auftragen, beispielsweise mit Pinsel oder Spraydosen oder durch Plasmavakuumbeschichtung.

Vorzugsweise besteht das Dichtelement aus einem elastomeren Material, welches mit keimhemmenden oder keimtötenden Mitteln vermischt ist. Beispielsweise besteht das Dichtelement aus Silikon mit beigemischten und/oder co-extrudierten keimhemmenden oder keimtötenden Mitteln. Beispielsweise besteht das Dichtelement aus Silikon mit integrierten oder beigemischten Silberionen.

Metallische Bestandteile der Dichtung sind vorzugsweise mit einer Legierung oder einer Dünnschicht versehen, welche keimhemmende oder keimtötende Mittel, wie beispielsweise Silber- und/oder Kupferverbindungen, aufweisen.

Vorzugsweise weist die erfindungsgemässe Dichtung zudem keine Materialien auf, in welche sich Keime bevorzugt anlagern. Vorzugsweise weist die Dichtung die in Absenkdichtungen üblicherweise verwendete Filzeinlage nicht auf. Diese Filzeinlage kann durch ein alternatives geeignetes Material ersetzt sein, welches ebenfalls Klappergeräusche des sich bewegenden Absenkmechanismus dämpft, welches jedoch Keimbildungen nicht unterstützt. Vorzugsweise ist eine derartige Einlage ebenfalls keimhemmend oder keimtötend ausgebildet. Sie kann beispielsweise mit einem geeigneten Material, insbesondere Silikon mit beigemischten oder integrierten Silberionen, bestehen oder mit einem derartigen Material beschichtet sein.

Weitere Ausführungsformen sind in den abhängigen Ansprüchen angegeben.

### KURZE BESCHREIBUNG DER ZEICHNUNGEN

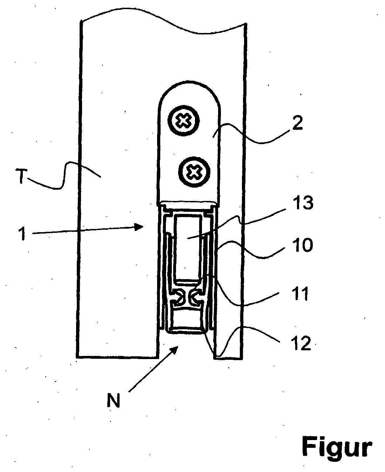

Bevorzugte Ausführungsformen der Erfindung werden im Folgenden anhand der Zeichnung beschrieben, die lediglich zur Erläuterung dient und nicht einschränkend auszulegen ist. Die einzige Figur zeigt eine Ansicht einer Türdichtung mit der erfindungsgemässen keimhemmenden oder keimtötenden Ausbildung.

### BESCHREIBUNG BEVORZUGTER AUSFÜHRUNGSFORMEN

Die einzige Figur zeigt eine Absenkdichtung 1, welche in einer unteren Nut N eines Türflügels T angeordnet ist. Die geometrische Ausbildung dieser Absenkdichtung 1 ist aus dem Stand der Technik bekannt. Sie ist vorzugsweise mittels Befestigungswinkeln 2, welche stirnseitig an beiden Enden der Absenkdichtung 1 in diese eingeschoben werden, am Türflügel befestigt. Sie kann tiefer versetzt in der Nut N angeordnet sein oder bündig mit der unteren Stirnseite des Türflügels enden. Im letzteren Fall lässt sie sich auch über ein Hutprofil an die untere Kante des Türflügels anschrauben. Andere Arten von Befestigungen sind ebenfalls möglich. Alternativ kann die Dichtung auch auf einer Stirnseite der Tür angeordnet sein oder an der unteren Stirnseite der Tür befestigt sein, ohne dass eine Nut in der Tür vorhanden sein muss. Auch derartige Anordnungen sind aus dem Stand der Technik bekannt.

Die Türdichtung 1 weist in diesem Beispiel ein Gehäuse 10 und eine darin angeordnete Trägerprofilschiene 11 auf. Gehäuse 10 und Trägerprofilschiene 11 sind in Form einer Profilschiene ausgebildet und bestehen vorzugsweise aus Aluminium oder Kunststoff. An der Trägerprofilschiene 11 ist ein ein- oder mehrstückiges Dichtelement 12 befestigt. Das Dichtelement 12 besteht vorzugsweise aus einem elastomeren oder anderweitig weichen und/oder elastischen Material.

In diesem Beispiel ist das Dichtelement 12 eine einstückige Dichtlippe, welche seitliche an der Trägerschiene 11 befestigt ist und dadurch einen unteren Bogen und zweite seitlich abstehende Schenkel ausbildet.

Die Trägerschiene 11 ist über einen nicht dargestellten Absenkmechanismus im Gehäuse 10 gehalten und gemeinsam mit dem Dichtelement 12 relativ zum Gehäuse 10 anhebbar und absenkbar. Derartige Absenkmechanismen sind aus dem Stand der Technik hinlänglich bekannt. Sie bestehen beispielsweise aus Blattfedern. Die Auslösung des Absenkmechanismus erfolgt vorzugsweise einseitig beim Schliessen und Öffnen des Türflügels, indem ein einseitig vorstehender federbelasteter Auslöseknopf eingedrückt wird und über eine Betätigungsstange auf die Blattfedern wirkt.

Im abgesenkten Zustand liegt der untere Bogen des Dichtelements 12 dichtend auf dem schwellenlosen Boden auf und dichtet somit den unteren Spalt des Türflügels T gegenüber dem Boden vor Lichtdurchlass, Schall und Zugluft ab. Die seitlichen Schenkel dichten vorzugsweise den Spalt zwischen Gehäuse 10 und Trägerschiene 11, so dass auch hier Zugluft und Schallübertragung vermieden wird.

In diesem Beispiel ist ein sich über einen Teil oder über die gesamte Länge der Dichtung erstreckendes quaderförmiges Dämpfungsprofil 13 vorhanden. Dieses dient zur Dämpfung der Geräusche, welche durch die Betätigung des Absenkmechanismus entstehen. Auf dieses Dämpfungsprofil kann auch verzichtet werden.

In der erfindungsgemässen Türdichtung ist nun mindestens ein Teil des Dichtelements keimhemmend oder keimtötend wirkend ausgebildet. Vorzugsweise ist das Dichtelement 12 aus einem derartigen Material hergestellt oder mit einem derartigen Material beschichtet. Es kann beispielsweise aus einem silberionenbeschichteten Silikon hergestellt sein. Es ist auch möglich, dass es lediglich mit einem silberionenbeschichteten Silikon beschichtet ist. Die Beschichtung ist in diesem Fall vorzugsweise beidseitig oder in einer anderen Ausführungsform mindestens auf der nach aussen gewandten Seite des Dichtelements. Es ist auch möglich, lediglich den unteren Bogen und/oder die seitlichen Schenkel des Dichtelements derartig keimhemmend oder keimtötend auszubilden.

In anderen Ausführungsformen ist zusätzlich mindestens das Gehäuse 10 und/oder die Trägerschiene 11 und/oder das Dämpfungsprofil 13 auf die oben beschrieben Art von keimhemmend oder keimtötend ausgebildet. Anstelle von Silikon lässt sich ein anderes geeignetes Material, insbesondere geeignete Beschichtungen verwenden. Die Beschichtung mit Silberionen enthaltendem Silikon oder anderem Kunststoff oder andere Arten von geeigneten Beschichtungen ist möglich.

Die Form der Gehäuses, der Trägerschiene sowie der Dichtleiste können auch anders ausgebildet sein als hier dargestellt. Beispiele hierfür sind hinlänglich aus dem Stand der Technik bekannt.

Die erfindungsgemässe Türdichtung erhöht die Hygiene in Gebäuden und ist insbesondere zur Verwendung in Spitälern und Kliniken geeignet.

**BEZUGSZEICHENLISTE**

| | | | |
|---|---|---|---|
| 1 | Türdichtung | 13 | Dämpfungsprofil |
| 10 | Gehäuse | 2 | Befestigungswinkel |
| 11 | Trägerschiene | T | Tür |
| 12 | Dichtelement | N | Nut |

## Patentansprüche

1. Türdichtung für eine schwellenlose Raumtür einer medizinischen Einrichtung, wobei die Türdichtung automatisch absenkbar ausgebildet ist und ein Dichtelement (12) zur Auflage auf einen Boden und zur Dichtung eines Spaltes zwischen Türflügel und Boden aufweist sowie ein Gehäuse (10), in welchem das Dichtelement (12) angeordnet ist, wobei das Dichtelement (12) relativ zum Gehäuse (10) automatisch anhebbar und absenkbar angeordnet ist, **dadurch gekennzeichnet, dass** mindestens ein Teil des Dichtelements (12) keimhemmend oder keimtötend wirkend ausgebildet ist, wobei dieser Teil dichtend auf dem Boden aufliegen kann.

2. Türdichtung nach Anspruch 1, wobei das gesamte Dichtelement (12) keimhemmend oder keimtötend wirkend ausgebildet ist.

3. Türdichtung nach Anspruch 1, wobei das Gehäuse (10) keimhemmend oder keimtötend wirkend ausgebildet ist.

4. Türdichtung nach einem der Ansprüche 1 bis 3, wobei das Dichtelement (12) an einer Trägerschiene (11) befestigt ist, welche im Gehäuse (10) gehalten ist.

5. Türdichtung nach Anspruch 4, wobei die Trägerschiene (11) keimhemmend oder keimtötend wirkend ausgebildet ist.

6. Türdichtung nach einem der Ansprüche 4 oder 5, wobei die Trägerschiene (11) gemeinsam mit dem Dichtelement (12) relativ zum Gehäuse (10) automatisch anhebbar und absenkbar ist.

7. Türdichtung nach einem der Ansprüche 1 bis 6, wobei die Absenkung und Anhebung über eine mechanische Auslösung erfolgt.

8. Türdichtung nach einem der Ansprüche 1 bis 7, wobei die gesamte Dichtung keimhemmend oder keimtötend wirkend ausgebildet ist.

9. Türdichtung nach einem der Ansprüche 1 bis 8, wobei die keimhemmende oder keimtötende Ausbildung eine Beschichtung und/oder Imprägnierung mit keimhemmenden oder keimtötenden Mitteln umfasst.

10. Türdichtung nach Anspruch 9, wobei die Beschichtung eine Beschichtung mit Silberionen ist.

11. Türdichtung nach Anspruch 1, wobei das Dichtelement (12) aus einem elastomeren Material besteht, welches mit keimhemmenden oder keimtötenden Mitteln vermischt ist.

12. Türdichtung nach Anspruch 1, wobei das Dichtelement (12) aus Silikon mit beigemischten und/oder co-extrudierten keimhemmenden oder keimtötenden Mitteln besteht.

13. Türdichtung nach Anspruch 1, wobei das Dichtelement (12) aus Silikon mit beigemischten oder integrierten Silberionen besteht.

## Claims

1. Door seal for a sill-free room door of a medical facility, wherein the door seal is automatically lowerable and has a sealing element (12) for bearing on a floor and for sealing a gap between the door leaf and the floor, and a housing (10) in which the sealing element (12) is arranged, wherein the sealing element (12) is automatically raisable and lowerable in relation to the housing (10), **characterized in that** at least part of the sealing element (12) is designed to have an antibacterial or bactericidal action, wherein this part can lie sealingly on the floor.

2. Door seal according to Claim 1, wherein the entire sealing element (12) is designed to have an antibacterial or bactericidal action.

3. Door seal according to Claim 1, wherein the housing (10) is designed to have an antibacterial or bactericidal action.

4. Door seal according to one of Claims 1 to 3, wherein the sealing element (12) is fastened to a carrier rail (11), which is held in the housing (10).

5. Door seal according to Claim 4, wherein the carrier rail (11) is designed to have an antibacterial or bactericidal action.

6. Door seal according to either of Claims 4 and 5, wherein the carrier rail (11) is automatically raisable and lowerable together with the sealing element (12) in relation to the housing (10).

7. Door seal according to either of Claims 1 and 6, wherein the lowering and raising takes place by mechanical triggering.

8. Door seal according to one of Claims 1 to 7, wherein the entire seal is designed to have an antibacterial or bactericidal action.

9. Door seal according to one of Claims 1 to 8, wherein the antibacterial or bactericidal design comprises using a coating and/or impregnation with antibacterial or bactericidal agents.

10. Door seal according to Claim 9, wherein the coating is a coating with silver ions.

11. Door seal according to Claim 1, wherein the sealing element (12) consists of an elastomeric material which is mixed with antibacterial or bactericidal agents.

12. Door seal according to Claim 1, wherein the sealing element (12) consists of silicone with admixed and/or co-extruded antibacterial or bactericidal agents.

13. Door seal according to Claim 1, wherein the sealing element (12) consists of silicone with admixed or integrated silver ions.

## Revendications

1. Joint de porte pour une porte d'un pièce sans seuil d'un établissement médical, dans lequel le joint de porte peut être abaissé automatiquement et présente un élément d'étanchéité (12) destiné à s'appliquer sur un sol et à obturer une fente entre un vantail de porte et un sol, ainsi qu'un boîtier (10), dans lequel l'élément d'étanchéité (12) est agencé, dans lequel l'élément d'étanchéité (12) peut être soulevé et abaissé automatiquement par rapport au boîtier (10), **caractérisé en ce qu'**au moins une partie de l'élément d'étanchéité (12) présente une activité antiseptique ou antibactérienne, cette partie pouvant reposer de manière étanche sur le sol.

2. Joint de porte selon la revendication 1, dans lequel tout l'élément d'étanchéité (12) présente une activité antiseptique ou antibactérienne.

3. Joint de porte selon la revendication 1, dans lequel le boîtier (10) présente une activité antiseptique ou antibactérienne.

4. Joint de porte selon l'une quelconque des revendications 1 à 3, dans lequel l'élément d'étanchéité (12) est fixé à un rail de support (11), qui est maintenu dans le boîtier (10).

5. Joint de porte selon la revendication 4, dans lequel le rail de support (11) présente une activité antiseptique ou antibactérienne.

6. Joint de porte selon l'une des revendications 4 ou 5, dans lequel le rail de support (11) peut être soulevé et abaissé automatiquement, en même temps que l'élément d'étanchéité (12), par rapport au boîtier (10).

7. Joint de porte selon l'une des revendications 1 ou 6, dans lequel le soulèvement et l'abaissement est effectué par un déclenchement mécanique.

8. Joint de porte selon l'une quelconque des revendications 1 à 7, dans lequel le joint entier présente une activité antiseptique ou antibactérienne.

9. Joint de porte selon l'une quelconque des revendications 1 à 8, dans lequel la formation antiseptique ou antibactérienne comprend un revêtement et/ou une imprégnation avec des agents antiseptiques ou antibactériens.

10. Joint de porte selon la revendication 9, dans lequel le revêtement est un revêtement avec des ions argent.

11. Joint de porte selon la revendication 1, dans lequel l'élément d'étanchéité (12) se compose d'un matériau élastomère, qui est mélangé à des agents antiseptiques ou antibactériens.

12. Joint de porte selon la revendication 1, dans lequel l'élément d'étanchéité (12) se compose de silicone avec des agents antiseptiques ou antibactériens ajoutés et/ou co-extrudés.

13. Joint de porte selon la revendication 1, dans lequel l'élément d'étanchéité (12) se compose de silicone avec des ions argent ajoutés ou intégrés.
